(19) Europäisches Patentamt
European Patent Office
Office européen des brevets

(11) EP 4 736 764 A2

(12) EUROPEAN PATENT APPLICATION

(43) Date of publication:
06.05.2026 Bulletin 2026/19

(21) Application number: 25210025.0

(22) Date of filing: 21.10.2025

(51) International Patent Classification (IPC):
A61B 5/20 $^{(2006.01)}$    A61B 5/00 $^{(2006.01)}$

(52) Cooperative Patent Classification (CPC):
A61B 5/204; A61B 5/208; A61B 5/6889;
A61B 5/6891; A61B 5/0059; A61B 5/205

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 30.10.2024 US 202463713852 P

(71) Applicant: Outsense Diagnostics Ltd.
Tel-Aviv 4791011 (IL)

(72) Inventor: KAPP-BARNEA, Yaara
4480500 Nirit (IL)

(74) Representative: Boult Wade Tennant LLP
Salisbury Square House
8 Salisbury Square
London EC4Y 8AP (GB)

(54) ASSESSING URINARY AND PELVIC-FLOOR FUNCTION

(57) Apparatus and methods are described, including one or more sensors (76) configured to sense a flow of urine from a subject's body, without contacting the subject's body, during a urination of the subject as the subject pauses the urination. A processor (44, 96) computes one or more parameters of the pause based on the sensing, and outputs a score based on the parameters. Other applications are also described.

EP 4 736 764 A2

## Description

### CROSS-REFERENCE TO RELATED APPLICATIONS

The present application claims priority from U.S. Provisional Patent Application No. 63/713,852 to Kapp-Barnea, filed October 30, 2024, entitled "Assessing Urinary and Pelvic-Floor Function," which is incorporated herein by reference.

### TECHNICAL FIELD

Embodiments relate generally to medical devices, and specifically to devices and methods for assessing urinary-system and pelvic-floor function, e.g., to facilitate improving such function by appropriate exercise.

### BACKGROUND

The urinary system includes the bladder, the urethra, and other organs. The bladder wall includes the detrusor muscle, which is continuous with the internal urethral sphincter. Both the detrusor muscle and the internal urethral sphincter are involuntary muscles.

The pelvic floor muscles, which include voluntary muscles such as the external urethral sphincter, support the pelvic organs and maintain continence. The pelvic floor muscles can be damaged or weakened due to injury, excessive weight gain, or, for women, life events such as hormonal changes, pregnancy, and vaginal delivery. This can lead to incontinence, constipation, genital prolapse, and other symptoms having a negative impact on quality of life.

### SUMMARY

Embodiments provide a system for computing a urination-related score. The system includes one or more sensors configured to sense a flow of urine from a subject during a urination of the subject, and a processor configured to compute the score based on the sensing performed by the sensors. The sensors do not contact the body of the subject; for example, in some embodiments, the sensors include a microphone and/or an imaging sensor mounted to the rim of the toilet bowl.

In some embodiments, the subject pauses the urination, e.g., in response to being prompted to do so via a smartphone application, and the sensors sense the flow of urine as the subject pauses the urination. Based on the sensing, the processor computes one or more parameters of the pause such as the duration required for the flow rate of the urine to decrease below a predefined threshold, a parameter quantifying a leakage of urine during the pause, the duration during which the flow rate is less than a predefined threshold, and/or an estimated force exerted by the subject's pelvic floor in pausing the flow. The processor then outputs a score, which in some embodiments indicates how well the subject's pelvic-floor muscles are functioning, based on the parameters.

In some embodiments, the subject does not necessarily pause the urination. Rather, the processor computes one or more parameters of the urination based on the sensing, and based on the parameters, outputs a score indicating the function of at least one involuntary muscle of the subject, such as the subject's detrusor muscle and/or internal urethral sphincter. For example, in some embodiments, the parameters include the delay between the arrival of the subject at the toilet and the start of the urination (a measure of urination hesitancy), the rate of increase of the flow rate at the start of the urination, and/or the duration during which the flow rate is close to its maximum.

There is therefore provided, in accordance with some embodiments, a system including one or more sensors configured to sense a flow of urine from a body of a subject, without contacting the body of the subject, during a urination of the subject as the subject pauses the urination, and a processor. The processor is configured to compute one or more parameters of the pause based on the sensing, and to output a score based on the parameters.

In some embodiments, the processor is further configured to prompt the subject to pause the urination.

In some embodiments, the sensors include a microphone configured to sense a sound of the flow.

In some embodiments, the sensors include an imaging sensor configured to image the flow.

In some embodiments, the urine flows into a toilet bowl, and the sensors are configured to couple to the toilet bowl.

In some embodiments, the processor is further configured to:

  receive an input indicating a measure of incontinence of the subject, and
  compute the score based on the measure of incontinence.

In some embodiments, the parameters include a duration required for a rate of the flow to decrease below a predefined threshold.

In some embodiments, the parameters include a rate of the flow prior to the subject pausing the urination.

In some embodiments, at least one of the parameters quantifies a leakage of the urine during the pause.

In some embodiments, the parameters include a duration during which a rate of the flow is less than a predefined threshold.

In some embodiments, the parameters include an estimated force exerted by a pelvic floor of the subject in pausing the flow.

In some embodiments, the processor is configured to compute the estimated force by:

  computing an estimated pressure applied, by the urine, to an external urethral sphincter of the subject, computing an estimated diameter of a urethra of the subject at the external urethral sphincter, and computing the estimated force based on the estimated pressure and the estimated diameter.

In some embodiments, the processor is configured to compute the estimated pressure by solving a Navier-Stokes equation describing the flow.

In some embodiments, the processor is configured to compute the estimated diameter of the urethra at the external urethral sphincter by:

based on the sensing, computing an estimated diameter of the urethra at an external boundary of the urethra, and

computing the estimated diameter of the urethra at the external urethral sphincter based on the estimated diameter of the urethra at the external boundary of the urethra.

There is further provided, in accordance with some embodiments, a method including, using one or more sensors that do not contact a body of a subject, sensing a flow of urine from the body of the subject, during a urination of the subject, as the subject pauses the urination. The method further includes, using a processor, based on the sensing, computing one or more parameters of the pause, and outputting a score based on the parameters.

There is further provided, in accordance with some embodiments, a method including, using one or more sensors that do not contact a body of a subject, sensing a flow of urine from the body of the subject during a urination of the subject. The method further includes, using a processor, based on the sensing, computing one or more parameters of the urination, and based on the parameters, outputting a score indicating a function of at least one involuntary muscle of the subject.

In some embodiments, the involuntary muscle is selected from the group of muscles including a detrusor muscle, and an internal urethral sphincter.

In some embodiments, the sensors include a microphone configured to sense a sound of the flow.

In some embodiments, the sensors include an imaging sensor configured to image the flow.

In some embodiments, the urine flows into a toilet bowl, and the sensors are coupled to the toilet bowl.

In some embodiments, the parameters include a delay between an arrival of the subject at a toilet and a start of the urination.

In some embodiments, the parameters include a rate of increase of a rate of the flow at a start of the urination.

In some embodiments, the parameters include a duration during which a difference between a maximum of a rate of the flow during the urination and the rate of the flow is less than a predefined threshold.

In some embodiments, the parameters include a duration during which, following an increase in a rate of the flow at a start of the urination, an absolute rate of change of the rate of the flow remains less than a predefined threshold.

The present disclosure will be more fully understood from the following detailed description of embodiments thereof, taken together with the drawings, in which:

## BRIEF DESCRIPTION OF THE DRAWINGS

Fig. 1 is a schematic illustration of a system for computing a urination-related score, in accordance with some embodiments;

Fig. 2 is a block diagram showing components of a sensor module, in accordance with some embodiments;

Fig. 3 and Fig. 4 show urination-related parameters calculated in accordance with some embodiments; and

Fig. 5 is a flow diagram for a method for improving pelvic-floor function, in accordance with some embodiments.

## DETAILED DESCRIPTION

SYSTEM DESCRIPTION

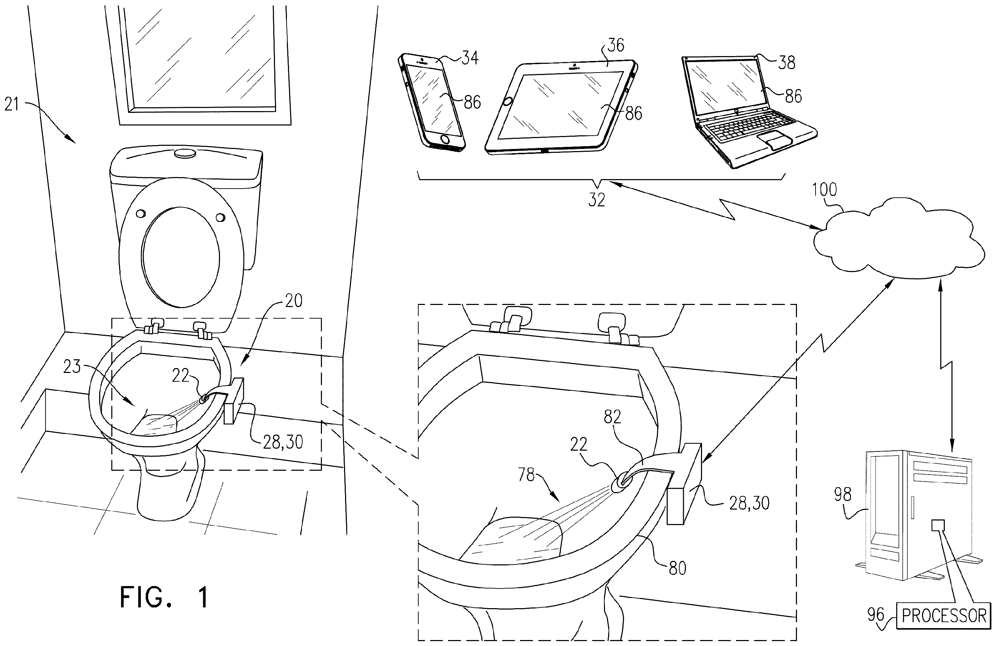

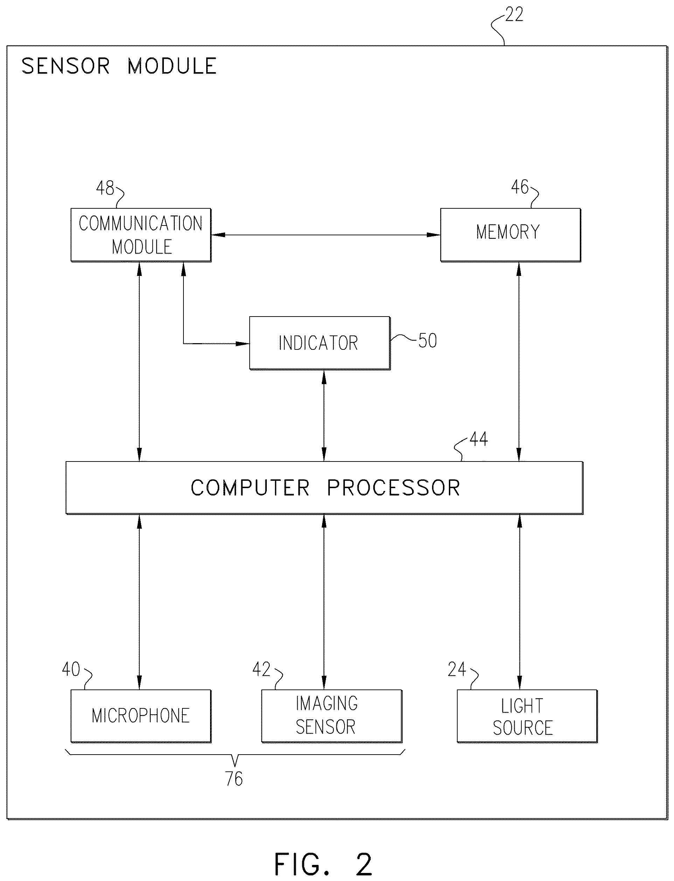

Reference is initially made to Fig. 1, which is a schematic illustration of a system 21 for computing a urination-related score, in accordance with some embodiments. Reference is also made to Fig. 2, which is a block diagram showing components of a sensor module 22, in accordance with some embodiments.

System 21 includes one or more sensors 76 (Fig. 2) configured to sense a flow of urine from the body of the subject, during a urination of the subject, passively, i.e., without contacting the body of the subject. In some examples, the subject urinates into a toilet bowl 23 of a toilet 20, and sensors 76 are coupled to the toilet bowl, such that the sensors sense the flow of urine as the urine flows into the toilet bowl. For example, in some examples, sensors 76 are contained in sensor module 22, which is integrated into toilet bowl 23 or mounted onto the rim 80 of toilet bowl 23. For example, in some embodiments, sensor module 22 is connected to a housing 30 by a connecting arm 82, which sits over rim 80 such that the sensor module is inside the toilet bowl and housing 30 is outside the toilet bowl. The sensor module can include a water-resistant housing. Housing 30 contains a power source 28 (e.g., a battery pack) configured to power sensor module 22 wiredly or wirelessly. Alternatively or additionally, sensor module 22 is connected to the mains electricity.

In some embodiments, sensors 76 include a microphone 40 configured to sense the sound of the flow. Alternatively or additionally, the sensors include an imaging sensor 42 configured to image the flow. In some such embodiments, system 21 (e.g., sensor module 22) further includes a light source 24 configured to emit light 78 at the urine as the urine flows from the subject's body, thereby illuminating the urine for imaging sensor 42. Alternatively or additionally, sensors 76 include an ultra-wideband sensor; for example, in some embodiments, system 21 (e.g., sensor module 22) further includes an ultra-wideband radar including an ultra-wideband emitter, which is configured to emit ultra-wideband waves at the flow, and an ultra-wideband sensor configured to detect reflections of the ultra-wideband waves. Alternatively or additionally, the sensors include an ultrasonic transducer configured to emit ultrasonic waves at the flow and to detect reflections of the ultrasonic waves.

In some embodiments, sensor module 22 includes a computer processor 44 configured to control, and/or receive signals from, other components of the sensor module such as light source 24 and/or sensors 76. In some examples, sensor module 22 further includes a communication module 48, including a wireless communication interface. In some embodiments, sensor module 22 further includes a memory 46 in which processor 44 is configured to store data. In some such embodiments, memory 46 includes a removable memory card, such as a Secure Digital card.

In some examples, as shown in Fig. 1, system 21 further includes a processor 96 configured to compute one or more parameters based on the sensing performed by sensors 76, i.e., based on the signals output by sensors 76 in response to the sensing. Processor 96 is further configured to output a quantitative or qualitative urination-related score for the subject based on the parameters.

In some embodiments, the urination-related score is one of the parameters. In other embodiments, processor 96 computes the urination-related score from the parameters, e.g., by inputting the parameters to a machine-learned model trained to output the urination-related score based on the parameters, or by applying a predefined closed-form function to the parameters. In some such embodiments, processor 96, in computing the urination-related score, maps the parameters to a number within a typical range of score values (e.g., 1-10) or to a qualitative description belonging to a typical set of such descriptions (e.g., the set of "very poor," "poor," "average," "good," and "very good").

In some embodiments, processor 96 belongs to a device 98, such as a cloud server, that is remote from toilet 20. In such embodiments, processor 44 of sensor module 22 is configured to communicate data received from sensors 76 over at least one network 100 (e.g., a Wi-Fi network and/or the Internet), to device 98, via communication module 48.

In some embodiments, system 21 is configured for use with at least one device 32 (e.g., a smartphone 34, a tablet computer 36, or a laptop computer 38) belonging, for example, to the subject or to the subject's healthcare provider. Processor 96 is configured to output the urination-related score to device 32, e.g., over network 100, and device 32 includes a display 86 configured to display the output. Alternatively or additionally, processor 96 outputs the urination-related score by storing the score in a memory and/or displaying the score on a display.

In some embodiments, one or more other processors perform at least some of the functionality of processor 96 described herein. For example, in some embodiments, processor 44 or a processor of device 32 computes the parameters by processing the data from sensors 76. Alternatively or additionally, for example, processor 44 or a processor of device 32 computes the urination-related score based on the parameters and/or outputs the score.

In some embodiments, sensor module 22 includes an indicator 50 configured to indicate to the subject when a urination has been successfully sensed, and/or when data has been successfully transmitted to a remote device. In some embodiments, indicator 50 includes a visual indicator, such as a light-emitting diode. Alternatively or additionally, indicator 50 includes an audio indicator (for example, a speaker that is configured to emit a beep).

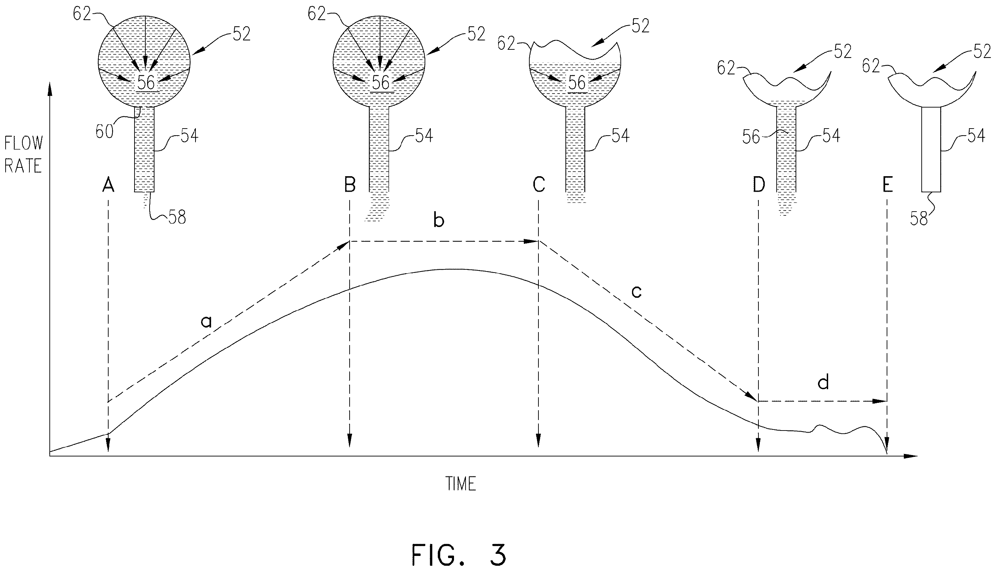

In some embodiments, processor 44 is further configured to process the signals from sensors 76 (e.g., microphone 40 and/or imaging sensor 42) so as to ascertain when the subject has arrived at the toilet. Alternatively or additionally, system 21 includes at least one additional sensor configured to output, to the processor, a signal indicating when the subject has arrived at the toilet. In some such embodiments, the additional sensor includes another passive sensor such as a proximity sensor (e.g., an infrared, ultrasonic, or ultra-wideband proximity sensor). In other such embodiments, the additional sensor includes a sensor coupled to the seat of toilet 20 and configured to output the signal in response to the subject contacting the sensor as the subject sits on the seat. Alternatively or additionally, the subject indicates her arrival at the toilet to the processor using an input interface, such as a button on or near the toilet or a virtual button in an application, such as an application running on smartphone 34. In response to ascertaining that the subject has arrived, the processor activates one or more components of system 21. Alternatively or additionally, the processor bases the urination-related score on the time of arrival, as further described below with reference to Fig. 3.

In general, each of the processors described herein may be embodied as a single processor, or as a cooperatively networked or clustered set of processors. The functionality of each processor may be implemented solely in hardware, e.g., using one or more fixed-function or general-purpose integrated circuits, Application-Specific Integrated Circuits, and/or Field-Programmable Gate Arrays. Alternatively, this functionality may be implemented at least partly in software. For example, the processor may be embodied as a programmed processor including, for example, a central processing unit and/or a graphics processing unit. Program code, including software programs, and/or data may be loaded for execution and processing by the central processing unit and/or graphics processing unit. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

OUTPUTTING THE URINATION-RELATED SCORE

Reference is now made to Fig. 3, which shows urination-related parameters calculated in accordance with some embodiments.

Fig. 3 schematically illustrates, for several points in time during a hypothetical urination of a subject, the bladder 52 and urethra 54 of the subject as urine 56 is expelled from bladder 52. In addition, Fig. 3 plots the flow rate of urine 56 as a function of time.

At time A, the subject voluntarily begins to open her external urethral sphincter 58. In response, the subject's internal urethral sphincter 60 begins to open involuntarily via a conditioned reflex, and simultaneously, via another conditioned reflex, the detrusor muscle 62 of bladder 52 begins to compress urine 56 from the bladder into urethra 54 by contracting inward. As the sphincters open and detrusor muscle 62 continues to compress the urine, the flow rate of urine increases during time period a, until the sphincters are fully open at time B. Urine 56 then continues to flow at close to its maximum rate during time period b, until time C. At time C, enough urine has been expelled such that detrusor muscle 62 has less contact with the urine in the bladder, and hence generates less pressure on the urine in the bladder. The flow rate of the urine therefore decreases, during the subsequent time period c, until time D. At time D, bladder 52 is almost completely empty, the subject begins to close external urethral sphincter 58, and internal urethral sphincter 60 begins to close involuntarily via a conditioned reflex. As the sphincters close, the flow rate decreases during time period d until, at time E, the flow of urine ceases.

In some embodiments, processor 96 (Fig. 1) computes one or more parameters of the urination based on the sensing performed by sensors 76 (Fig. 2). Based on the parameters, the processor outputs a urination-related score indicating the function of at least one involuntary muscle (e.g., detrusor muscle 62 and/or internal urethral sphincter 60) of the subject. In other words, the score indicates how well the at least one involuntary muscle is functioning.

For example, in some embodiments, the parameters include the delay between the arrival of the subject at the toilet and the start of the urination (which is at around time A), the delay being a measure of urination hesitancy. Based on this parameter, the processor outputs a score indicating the health of the conditioned reflex of detrusor muscle 62 and/or internal urethral sphincter 60.

Alternatively or additionally, in some embodiments, the parameters include the rate of increase of the flow rate at the start of the urination, e.g., during a period approximately corresponding to time period a. Based on this parameter, the processor outputs a score indicating the flexibility and/or strength of detrusor muscle 62, the flexibility of internal urethral sphincter 60, and/or the health of the conditioned reflex of detrusor muscle 62 and/or internal urethral sphincter 60.

Alternatively or additionally, the parameters include the duration during which the difference between the maximum flow rate and the flow rate is less than a predefined threshold, and/or the duration during which, following the increase in the flow rate at the start of the urination, the absolute rate of change of the flow rate remains less than a predefined threshold. In some examples, each of these durations approximately corresponds to time period b. Based on one or both of these parameters, the processor outputs a score indicating the flexibility, force with maximal load, and/or elasticity of detrusor muscle 62.

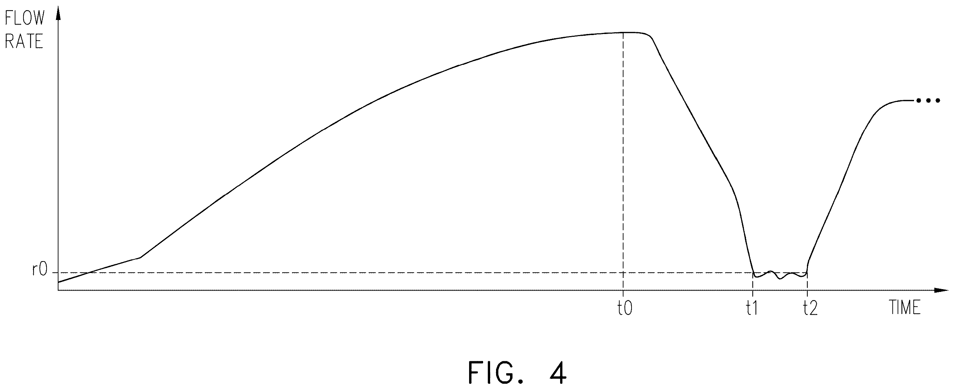

Alternatively, the urination-related score is indicative of the function of one or more voluntary muscles, such as the pelvic floor muscles, and/or the function of other components of the urinary system. In this regard, reference is now made to Fig. 4, which shows urination-related parameters calculated in accordance with some embodiments.

In some embodiments, at a time t0 during the subject's urination, the subject initiates a pause in the urination. In some examples, the pause is initiated during time period b (Fig. 3), when the flow rate of the urine is at or near its maximum.

For example, in some embodiments, the processor prompts the subject, at time t0, to pause the urination, e.g., by driving an application running on device 32 (Fig. 1) to prompt the subject by outputting a visual and/or an audio prompt. For example, in some embodiments, the processor prompts the subject in response to the flow rate exceeding a predefined threshold for a predefined amount of time (e.g., 1-5 s), and/or in response to the rate of change of the flow rate being less than a predefined threshold for a predefined amount of time (e.g., 1-5 s). In other embodiments, the subject initiates the pause even without being prompted. For example, in some embodiments, the subject initiates the pause in response to a subjective feeling that a maximum flow rate has been sustained for several seconds.

In some embodiments, the subject is prompted, or the subject attempts even without being prompted, to pause the flow of urine for at least a predefined minimal amount of time, such as 1-5 s. Alternatively or additionally, the subject is notified that the duration of the pause need not exceed a predefined maximum value. In other embodiments, the target duration of the pause is left to the subject's discretion.

Sensors 76 (Fig. 2) sense the flow of urine as the subject pauses the urination. Based on the sensing, processor 96 (Fig. 1) computes one or more parameters of the pause. The processor then outputs the urination-related score based on these parameters.

For example, in some embodiments, assuming the subject succeeds in decreasing the flow rate below a predefined threshold r0 at time t1, the parameters include the duration required for the flow rate to decrease below r0 from the flow rate prior to the pause (which, as noted above, may be at or near the maximal flow rate during the urination). For example, this parameter can be computed as t1 - t0. (For embodiments in which the subject is not prompted to initiate the pause, t0 can be estimated, e.g., by subtracting a predefined amount of time from the time at which the flow rate begins to decrease.) This parameter, which may be referred to as the "urine-arrest duration" of the subject, indicates the strength of the pelvic-floor muscles and/or the speed of neurofeedback to the pelvic-floor muscles. In some examples, the score is higher for a shorter duration, relative to a longer duration.

Alternatively or additionally, the parameters include the flow rate of the urine prior to the subject pausing the urination, e.g., the flow rate at t0. In some examples, the score is higher for a greater initial flow rate, relative to a lesser initial flow rate.

Alternatively or additionally, at least one of the parameters quantifies the leakage of urine during the pause. For example, in some embodiments, "leakage" is defined as any flow of urine, during the pause, with a flow rate that is less than a predefined threshold, which can be r0 or another value greater than or less than r0. To quantify the leakage, the processor can compute, for example, the total amount of leaked urine and/or the duration of the leakage. In some examples, the score is higher for less leakage, relative to more leakage.

Alternatively or additionally, the parameters include the duration for which the flow rate is less than a predefined threshold, which can be r0 or another value greater than or less than r0. For example, assuming the flow rate remains less than r0 until time t2, the parameter can be computed as t2 - t1. In some examples, the score is higher for a longer duration, relative to a shorter duration.

Alternatively or additionally, the parameters include the estimated force exerted by the pelvic floor of the subject in pausing the flow. For example, in some embodiments, the processor computes the estimated pressure applied, by the urine, to external urethral sphincter 58 (Fig. 3), along with the estimated diameter of urethra 54 (Fig. 3) at the external urethral sphincter. Next, the processor computes the estimated force based on the estimated pressure and the estimated diameter. In some examples, the score is higher for a greater estimated force, relative to a lesser estimated force.

In some embodiments, the processor computes the estimated pressure by solving the Navier-Stokes equation describing the flow, under the assumption that the urine flow in the urethra is laminar and Newtonian, and based on predefined values for the density and viscosity of the urine. The Navier-Stokes equation is as follows:

$$\rho\left(\frac{\partial v}{\partial t} + v \cdot \nabla v\right) = -\nabla p + \mu \nabla^2 v + F, \nabla \cdot v = 0 \quad ,$$

where $\rho$ is the urine density, v is the velocity vector of the flow (which is derived from the sensing of the flow), p is the pressure (for which the processor solves the equation), $\mu$ is the urine viscosity, and F is the force vector for gravity and any other external forces.

Alternatively or additionally, to compute the estimated diameter of the urethra at the external urethral sphincter, the processor first computes the estimated diameter of the urethra at the external boundary of the urethra, based on the sensing of the flow. Next, the processor computes the estimated diameter of the urethra at the external urethral sphincter based on the estimated diameter of the urethra at the external boundary of the urethra, e.g., using a differential equation solver such as Ansys Fluent™ or COMSOL Multiphysics™.

In some embodiments, the subject inputs a measure of incontinence of the subject, such as an estimated frequency of involuntary urination, to processor 96, and the processor computes the score based on the measure of incontinence. In some examples, the score is higher for less incontinence, relative to more incontinence.

In some embodiments, based on the sensing performed by sensors 76 (Fig. 2), processor 96 computes the volumes of urine expelled during one or more urinations of the subject. Based on the computed volumes, the processor computes and outputs an estimated volume of the subject's bladder. For example, in some embodiments, after computing the expelled urine volumes for several urinations, the processor estimates the bladder volume based on the maximum of the urine volumes, e.g., by setting the bladder-volume estimate equal to this maximum. Alternatively or additionally, the processor estimates the bladder volume based on input from the subject, such as an input, for each urination, indicating the subject's estimate of how full the bladder was at the start of the urination.

## PELVIC-FLOOR TRAINER

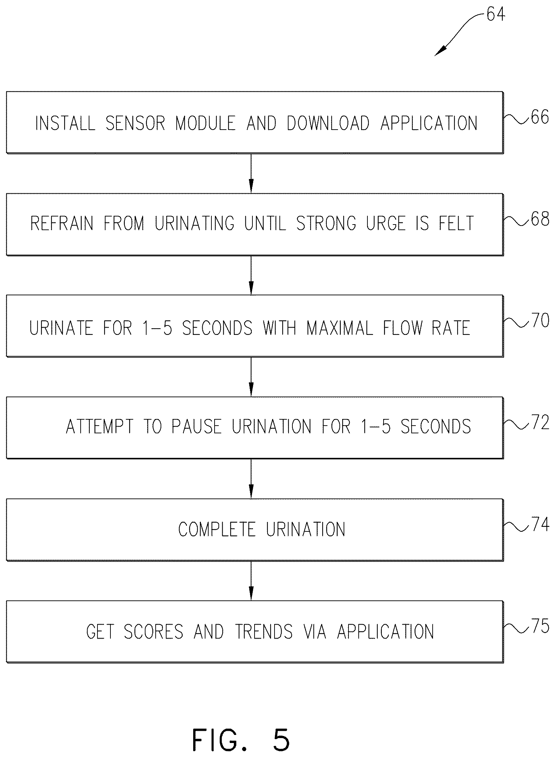

Reference is now made to Fig. 5, which is a flow diagram for a method 64 for improving pelvic-floor function, in accordance with some embodiments.

In some embodiments, at a setup step 66, the subject installs sensor module 22 (Fig. 1) and downloads a pelvic-floor trainer application to device 32 (Fig. 1). Next, each time the subject wishes to train her pelvic floor, the subject refrains from urinating, at a refraining step 68, until a strong urge to urinate is felt. The strong urge generally provides a more intensive exercise for the subject's pelvic floor.

Once a strong urge is felt, the subject urinates, e.g., for 1-5 seconds with a maximal flow rate, at a first urinating step 70. The subject then attempts to pause the urination, e.g., for 1-5 seconds, at a pausing step 72. Following the pause (or if the attempt was unsuccessful), the subject completes the urination at a second urinating step 74. Finally, the subject gets any urination-related scores, and optionally personal and/or population-wide trends in the scores, via the application, at an output-receiving step 75. In this manner, the application functions as a wellness biofeedback tool, allowing the subject to track her progress over time as the subject exercises to improve her scores.

It will be appreciated by persons skilled in the art that the present disclosure is not limited to what has been particularly shown and described hereinabove. Rather, the scope includes both combinations and subcombinations of the various features described hereinabove, as well as variations and modifications thereof that are not in the prior art, which would occur to persons skilled in the art upon reading the foregoing description.

**Claims**

1. A system, comprising:

   one or more sensors (76) configured to sense a flow of urine from a body of a subject, without contacting the body of the subject, during a urination of the subject as the subject pauses the urination; and
   a processor (44, 96), configured to:

   compute one or more parameters of the pause based on the sensing, and
   output a score based on the parameters.

2. The system according to claim 1, wherein the processor (44, 96) is further configured to prompt the subject to pause the urination.

3. The system according to claim 1, wherein the sensors (76) comprise a microphone (40) configured to sense a sound of the flow, and/or an imaging sensor (42) configured to image the flow.

4. The system according to claim 1, wherein the urine flows into a toilet bowl (23), and wherein the sensors (76) are configured to couple to the toilet bowl.

5. The system according to claim 1, wherein the processor (44, 96) is further configured to:

   receive an input indicating a measure of incontinence of the subject, and
   compute the score based on the measure of incontinence.

6. The system according to claim 1, wherein the parameters include a duration required for a rate of the flow to decrease below a predefined threshold, a rate of the flow prior to the subject pausing the urination, a duration during which a rate of the flow is less than a predefined threshold.

7. The system according to claim 1, wherein at least one of the parameters quantifies a leakage of the urine during the pause.

8. The system according to any one of claims 1-7, wherein the parameters include an estimated force exerted by a pelvic floor of the subject in pausing the flow.

9. The system according to claim 8, wherein the processor (44, 96) is configured to compute the estimated force by:

   computing an estimated pressure applied, by the urine, to an external urethral sphincter of the subject,
   computing an estimated diameter of a urethra of the subject at the external urethral sphincter, and
   computing the estimated force based on the estimated pressure and the estimated diameter.

10. The system according to claim 9, wherein the processor (44, 96) is configured to compute the estimated diameter of the urethra at the external urethral sphincter by:

   based on the sensing, computing an estimated diameter of the urethra at an external boundary of the urethra, and
   computing the estimated diameter of the urethra at the external urethral sphincter based on the estimated diameter of the urethra at the external boundary of the urethra.

11. A method, comprising:

   using one or more sensors (76) that do not contact a body of a subject, sensing a flow of urine from the body of the subject, during a urination of the subject, as the subject pauses the urination; and

using a processor (44, 96):

> based on the sensing, computing one or more parameters of the pause, and outputting a score based on the parameters.

12. The method according to claim 11, further comprising:

> receiving, by the processor (44, 96), an input indicating a measure of incontinence of the subject; and
>
> computing the score based on the measure of incontinence.

13. The method according to claim 11, wherein the parameters include a duration required for a rate of the flow to decrease below a predefined threshold, a rate of the flow prior to the subject pausing the urination, and/or a duration during which a rate of the flow is less than a predefined threshold.

14. The method according to claim 11, wherein at least one of the parameters quantifies a leakage of the urine during the pause.

15. The method according to any one of claims 11-14, wherein the parameters include an estimated force exerted by a pelvic floor of the subject in pausing the flow.

FIG. 1

SENSOR MODULE
22

48
COMMUNICATION MODULE

46
MEMORY

50
INDICATOR

44
COMPUTER PROCESSOR

40
MICROPHONE

42
IMAGING SENSOR

24
LIGHT SOURCE

76

FIG. 2

FIG. 3

EP 4 736 764 A2

FIG. 4

64

INSTALL SENSOR MODULE AND DOWNLOAD APPLICATION — 66

REFRAIN FROM URINATING UNTIL STRONG URGE IS FELT — 68

URINATE FOR 1–5 SECONDS WITH MAXIMAL FLOW RATE — 70

ATTEMPT TO PAUSE URINATION FOR 1–5 SECONDS — 72

COMPLETE URINATION — 74

GET SCORES AND TRENDS VIA APPLICATION — 75

FIG. 5

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63713852, Kapp-Barnea